# EUROPEAN PATENT SPECIFICATION

(11) **EP 1 781 186 B1**
(45) Date of publication and mention of the grant of the patent: **13.03.2013**
(21) Application number: 05779614.6
(22) Date of filing: 04.08.2005
(51) Int. Cl.: A61B 17/12

(54) **VENTRICULAR PARTITIONING DEVICE**
VENTRIKELUNTERTEILUNGSVORRICHTUNG
DISPOSITIF DE SEPARATION VENTRICULAIRE

(30) Priority: 05.08.2004 US 913608
(43) Date of publication of application: 09.05.2007
(73) Proprietor: Cardiokinetix, Inc., Menlo Park, CA 94025 (US)
(72) Inventor: SHARKEY, Hugh, R., Redwood City, CA 94062 (US); KHAIRKHAHAN, Alexander, Palo Alto, CA 94302 (US); NIKOLIC, Serjan, D., San Francisco, CA 94121 (US); RADOVANCEVIC, Branislav, Houston, TX 77005 (US)
(74) Representative: Price, Nigel John King
(86) International application number: PCT/US2005/028065
(87) International publication number: WO 2006/017809

(56) References cited:
- WO-A-02/071977
- WO-A-2004/100803
- US-A- 6 152 144
- US-A1- 2003 120 337

## Description

### FIELD OF THE INVENTION

The present invention relates generally to the field of treating congestive heart failure and more specifically, to a device for partitioning a patents heart chamber.

### BACKGROUND OF THE INVENTION

Congestive heart failure (CHF) is characterized by a progressive enlargement of the heart, particularly the left ventricle and is a major cause of death and disability in the United States. Approximately 500,000 cases occur annually in the U.S. alone. As the patient's heart enlarges, it cannot efficiently pump blood forward with each heart beat. In time, the heart becomes so enlarged the heart cannot adequately supply blood to the body. Even in healthy hearts only a certain percentage of the blood in a patient's left ventricle is pumped out or ejected from the chamber during each stroke of the heart. The pumped percentage, commonly referred to as the "ejection fraction", is typically about sixty percent for a healthy heart. A patient with congestive heart failure can have an ejection fraction of less than 40% and sometimes lower. As a result of the low ejection fraction, a patient with congestive heart failure is fatigued, unable to perform even simple tasks requiring exertion and experiences pain and discomfort. Further, as the heart enlarges, the internal heart valves such as the mitral valve, cannot adequately close. An incompetent mitral valve allows regurgitation of blood from the left ventricle back into the left atrium, further reducing the heart's ability to pump blood forewardly.

Congestive heart failure can result from a variety of conditions, including viral infections, incompetent heart valves (e.g. mitral valve), ischemic conditions in the heart wall or a combination of these conditions. Prolonged ischemia and occlusion of coronary arteries can result in myocardial tissue in the ventricular wall dying and becoming scar tissue. Once the myocardial tissue dies, it is less contractile (sometimes non-contractile) and no longer contributes to the pumping action of the heart. It is referred to as hypokinetic. As the disease progresses, a local area of compromised myocardium may bulge out during the heart contractions, further decreasing the heart's ability to pump blood and further reducing the ejection fraction. In this instance, the heart wall is referred to as dyskinetic or akinetic. The dyskinetic region of the heart wall may stretch and eventually form an aneurysmic bulge.

Patients suffering from congestive heart failure are commonly grouped into four classes, Classes I, II, III and IV. In the early stages, Classes I and II, drug therapy is presently the most commonly prescribed treatment. Drug therapy typically treats the symptoms of the disease and may slow the progression of the disease, but it can not cure the disease. Presently, the only permanent treatment for congestive heart disease is heart transplantation, but heart transplant procedures are very risky, extremely invasive and expensive and are performed on a small percentage of patients. Many patient's do not qualify for heart transplant for failure to meet any one of a number of qualifying criteria, and, furthermore, there are not enough hearts available for transplant to meet the needs of CHF patients who do qualify.

Substantial effort has been made to find alternative treatments for congestive heart disease. For example, surgical procedures have been developed to dissect and remove weakened portions of the ventricular wall in order to reduce heart volume. This procedure is highly invasive, risky and expensive and is commonly only done in conjunction with other procedures (such as heart valve replacement or coronary artery by-pass graft). Additionally, the surgical treatment is usually limited to Class IV patients and, accordingly, is not an option for patients facing ineffective drug treatment prior to Class IV. Finally, if the procedure fails, emergency heart transplant is the only presently available option.

Other efforts to treat CHF include the use of an elastic support, such as an artificial elastic sock placed around the heart to prevent further deleterious remodeling.

Additionally, mechanical assist devices have been developed as intermediate procedures for treating congestive heart disease. Such devices include left ventricular assist devices and total artificial hearts. A left ventricular assist device includes a mechanical pump for increasing blood flow from the left ventricle into the aorta. Total artificial heart devices, such as the Jarvik heart, are usually used only as temporary measures while a patient awaits a donor heart for transplant.

Recently, improvements have been made in treating patient's with CHF by implanting pacing leads in both sides of the heart in order to coordinate the contraction of both ventricles of the heart. This technique has been shown to improve hemodynamic performance and can result in increased ejection fraction from the right ventricle to the patient's lungs and the ejection fraction from the left ventricle to the patient's aorta. While this procedure has been found to be successful in providing some relief from CHF symtoms and slowed the progression of the disease, it has not been able to stop the disease.

US 2003/120337 A1 discloses a membrane applied to the ostium of an atrial appendage for blocking blood from entering the atrial appendage which can form blood clots therein is disclosed. The membrane also prevents blood clots in the atrial appendage from escaping therefrom and entering the blood stream which can result in a blocked blood vessel, leading to strokes and heart attacks. The membranes are percutaneously installed in patients experiencing atrial fibrillations and other heart conditions where thrombosis may form in the atrial appendages. A variety of means for securing the membranes in place are disclosed. The membranes may be held in place over the ostium of the atrial appendage or fill the inside of the atrial appendage. The means for holding the membranes in place over the ostium of the atrial appendages include prongs, stents, anchors with tethers or springs, disks with tethers or springs, umbrellas, spiral springs filling the atrial appendages, and adhesives. After the membrane is in place a filler substance may be added inside the atrial appendage to reduce the volume, help seal the membrane against the ostium or clot the blood in the atrial appendage. The membranes may have anticoagulants to help prevent thrombosis. The membranes be porous such that endothelial cells cover the membrane presenting a living membrane wall to prevent thrombosis. The membranes may have means to center the membranes over the ostium. Sensors may be attached to the membrane to provide information about the patient.

### SUMMARY OF THE INVENTION

According to the present invention there is provided the device of claim 1.

The hereinafter described and illustrated preferred embodiment of device is a ventricular partitioning device for use in the treatment of a patient with congestive heart failure (CHF). Specifically, the preferred embodiment of device partitions a chamber of the patient's heart into a main productive portion and a secondary non-productive portion. This partitioning reduces the total volume of the heart chamber, reduces the stress applied to the heart and, as a result, improves the ejection fraction thereof.

A partitioning device embodying features of the invention has a reinforced partitioning component with a concave, pressure receiving surface which defines in part the main productive portion of the partitioned heart chamber when secured within the patient's heart chamber.

The reinforced partitioning component preferably includes a hub and a membrane forming the pressure receiving surface. The partitioning component is reinforced by a radially expandable frame component formed of a plurality of ribs.

The ribs of the expandable frame have distal ends secured to the central hub and free proximal ends. The distal ends are preferably secured to the central hub to facilitate radial self expansion of the free proximal ends of the ribs away from a centerline axis. The distal ends of the ribs may be pivotally mounted to the hub and biased outwardly or fixed to the hub and formed of material such as superelastic NiTi alloy which allows for compressing the free proximal ends of the ribs toward a centerline axis into a contracted configuration and when released allow for their self expansion to an expanded configuration.

The free proximal ends of the ribs are configured to engage and preferably penetrate the tissue lining the heart chamber to be partitioned so as to secure the peripheral edge of the partitioning component to the heart wall and fix the partitioning component within the chamber so as to partition the chamber in a desired manner. The tissue penetrating proximal tips are configured to penetrate the tissue lining at an angle approximately perpendicular to a center line axis of the partitioning device. The tissue penetrating proximal tips of the ribs may be provided with barbs, hooks and the like which prevent withdrawal from the tips from the heart wall.

The ribs in their expanded configuration angle outwardly from the hub and the free proximal ends curve outwardly so that the membrane secured to the ribs of the expanded frame forms a trumpet-shaped, pressure receiving surface.

The partitioning membrane in the expanded configuration has radial dimensions from about 10 to about 160 mm, preferably about 50 to about 100 mm, as measured from the center line axis.

The partitioning device may be delivered percutaneously or intraoperatively. One particularly suitable delivery catheter has an elongated shaft, a releasable securing device on the distal end of the shaft for holding the partitioning device on the distal end and an expandable member such as an inflatable balloon on a distal portion of the shaft proximal to the distal end to press the interior of the recess formed by the pressure receiving surface to ensure that the tissue penetrating tips or elements on the periphery of the partitioning device penetrate sufficiently into the heart wall to hold the partitioning device in a desired position to effectively partition the heart chamber.

The partitioning device embodying features of the invention is relatively easy to install and it substantially improves the pumping action of the heart and provides an increase in the ejection fraction of the patient's heart chamber. These and other advantages of the invention will become more apparent from the following detailed description of the invention and the accompanying exemplary drawings.

### BRIEF DESCRIPTION OF THE DRAWINGS

Figure 1 is an elevational view of a partitioning device embodying features of the invention in an expanded configuration.

Figure 2 is a plan view of the partitioning device shown in Figure 1.

Figure 3 is a partial longitudinal cross-sectional view of the hub of the partitioning device shown in Figure 1.

Figure 4 is a transverse cross sectional view of the hub shown in Figure 3 taken along the lines 4-4.

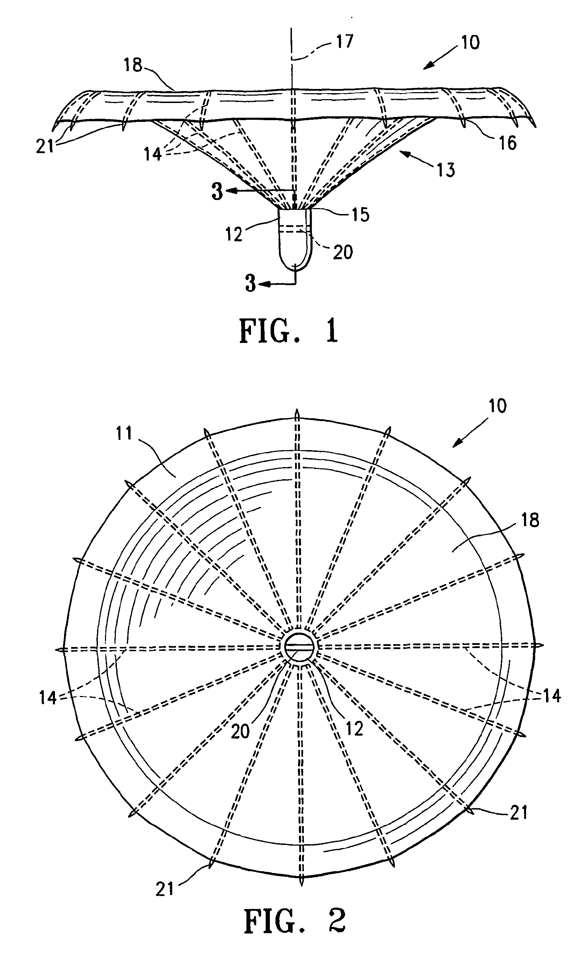

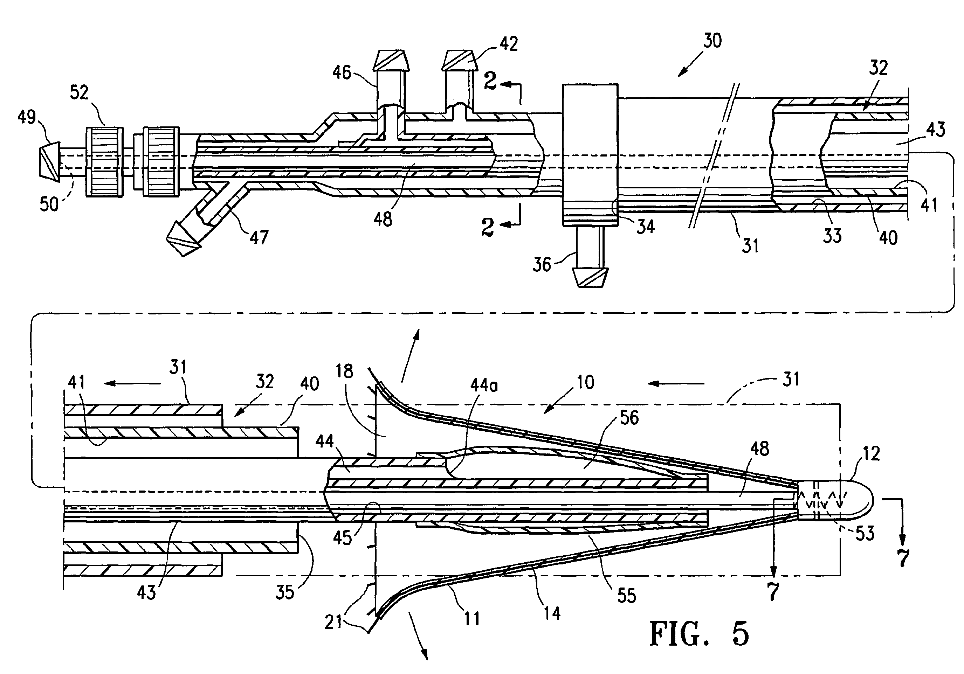

Not forming part of the invention, in figure 5 is a schematic elevational view of a delivery system for the partitioning device shown in Figures 1 and 2.

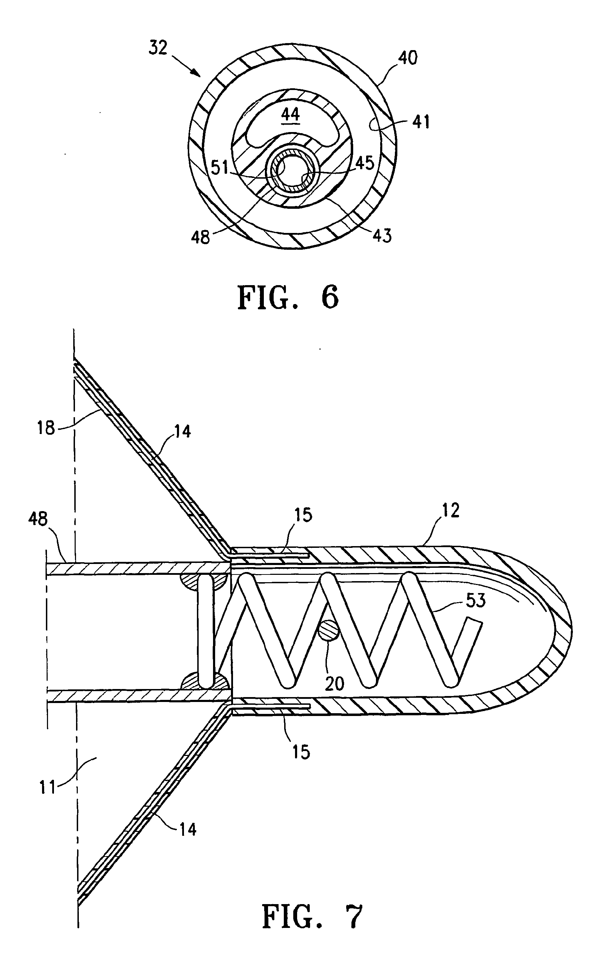

Figure 6 is a transverse cross-sectional view of the delivery system shown in Figure 5 taken along the lines 6-6.

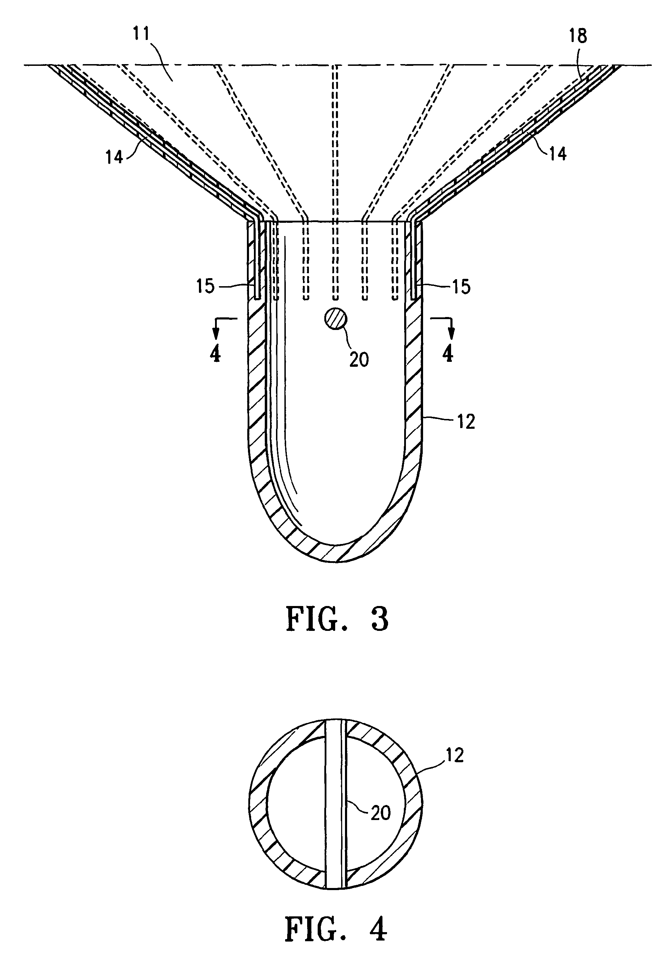

Figure 7 is an elevational view, partially in section, of the hub shown in Figure 3 secured to the helical coil of the delivery system shown in Figure 5.

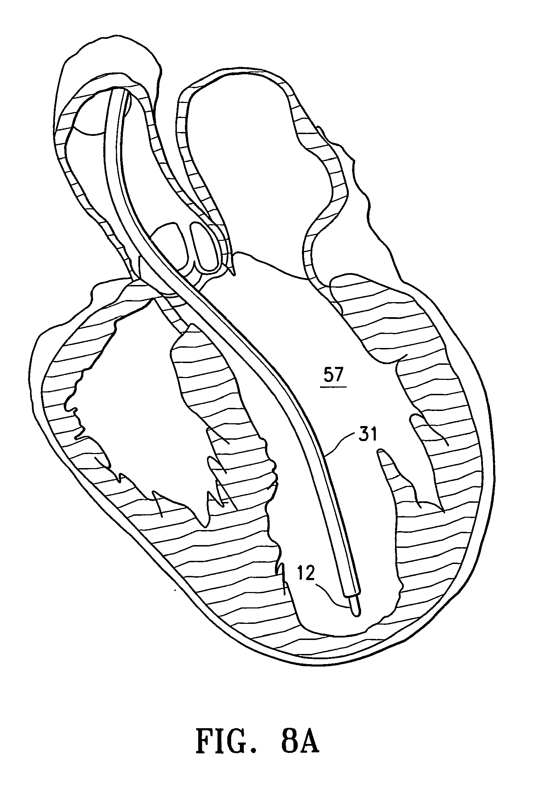

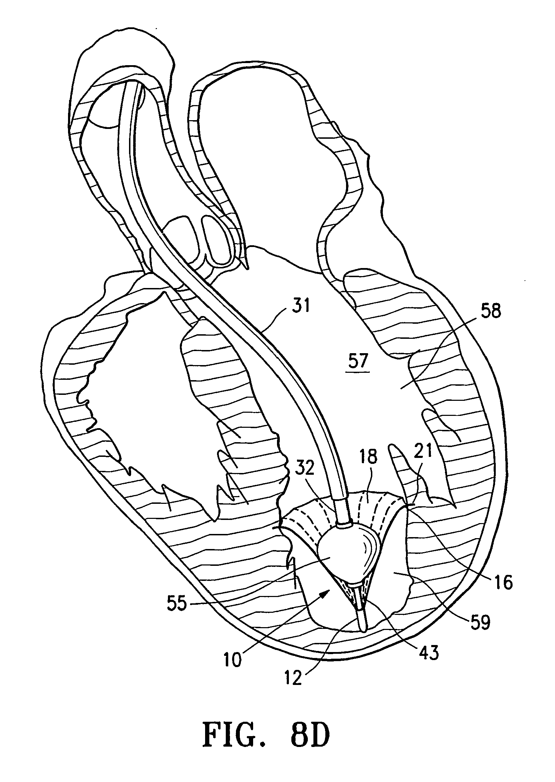

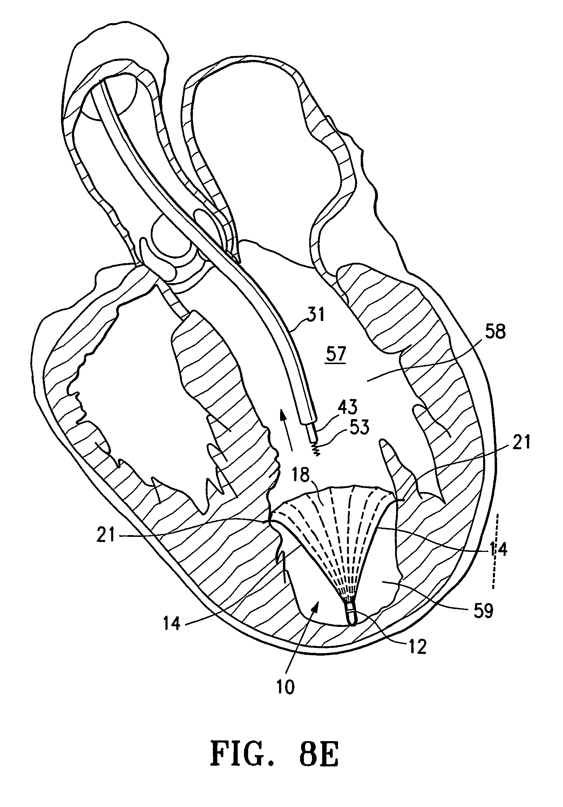

Figures 8A-8E are schematic views of a patient's left ventricular chamber illustrating the deployment of the partitioning device shown in Figures 1 and 2 with the delivery system shown in Figure 5 to partition the heart chamber into a primary productive portion and a secondary, non-productive portion.

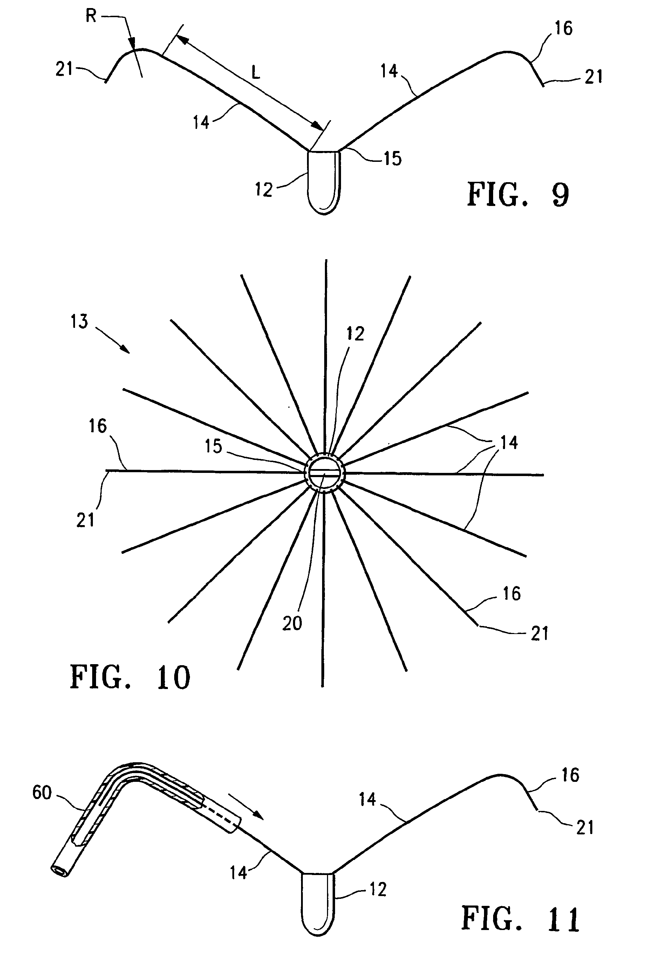

Figure 9 is a partial schematic view of the expandable frame of the partitioning device shown in Figures 1 and 2 in an unrestricted configuration.

Figure 10 is a top view of the expandable frame shown in Figure 9.

Not forming part of the invention, in figures 11-13 are schematic illustrations of a method or forming the partitioning device shown in Figures 1 and 2 from the expandable frame shown in Figures 9 and 10.

### DETAILED DESCRIPTION OF EMBODIMENTS OF THE INVENTION

Figures 1-4 illustrate a partitioning component 10 which embodies features of the invention and which includes a partitioning membrane 11, a hub 12, preferably centrally located on the partitioning device, and a radially expandable reinforcing frame 13 formed of a plurality of ribs 14. Preferably, the partitioning membrane 11 is secured to the proximal or pressure side of the frame 13 as shown in Figure 1. The ribs 14 have distal ends 15 which are secured to the hub 12 and free proximal ends 16 which are configured to curve or flare away from a center line axis 17. Radial expansion of the free proximal ends 16 unfurls the membrane 11 secured to the frame 13 so that the membrane presents a relatively smooth, pressure receiving surface 18 which defines in part the productive portion of the patient's partitioned heart chamber.

As shown in more detail in Figures 3 and 4, the distal ends 15 of the ribs 14 are secured within the hub 12 and a transversely disposed connector bar 20 is secured within the hub which is configured to secure the hub 12 and thus the partitioning component 10 to a delivery system such as shown in Figure 5 and 6. The curved free proximal ends 16 of ribs 14 are provided with sharp tip elements 21 which are configured to hold the frame 13 and the membrane 11 secured thereto in a deployed position within the patient's heart chamber. Preferably, the sharp tip elements 21 of the frame 13 penetrate into tissue of the patient's heart wall in order to secure the partitioning component 10 within the heart chamber so as to partition the ventricular chamber into a productive portion and a non-productive portion.

The connector bar 20 of the hub 12, as will be described later, allows the partitioning device 10 to be secured to a delivery system delivery and to be released from the delivery system within the patient's heart chamber. The distal ends 15 of the reinforcing ribs 14 are secured within the hub 12 in a suitable manner or they may be secured to the surface defining the inner lumen or they may be disposed within channels or bores in the wall of the hub 12. The ribs 14 are preshaped so that when not constrained other than by the membrane 11 secured thereto (as shown in Figures 1 and 2), the free proximal ends 16 thereof expand to a desired angular displacement away from a center line axis 17 which is about 20° to about 90°, preferably about 50° to about 80°.

Figures 5-7 illustrate a suitable delivery system 30 not forming part of the invention delivering the partitioning component 10 shown in Figures 1 and 2 into a patient's heart chamber and deploying the partitioning component 10 to partition the heart chamber as shown in Figures 8A - 8E. The delivery system 30 includes a guide catheter 31 and a delivery catheter 32.

The guide catheter has an inner lumen 33 extending between the proximal end 34 and distal end 35. A hemostatic valve (not shown) may be provided at the proximal end 34 of the guide catheter 31. A flush port 36 on the proximal end 34 of guide catheter 31 is in fluid communication with the inner lumen 33.

The delivery catheter 32 has an outer shaft 40 with an inner lumen 41 and a proximal injection port 42, an inner shaft 43 disposed within the inner lumen 41 with a first lumen 44 and a second lumen 45. Balloon inflation port 46 is in fluid communication with the first lumen 44 and flush port 47 is in fluid communication with the inner lumen 41. Torque shaft 48 is rotatably disposed within the second lumen 45 of the inner shaft 43 and has an injection port 49 provided at its proximal end 50 in fluid communication with the inner lumen 51 of the torque shaft The torque shaft 48 is preferably formed at least in part of a hypotube formed of suitable material such as superelastic NITINOL or stainless steel. A torque knob 52 is secured to the proximal end 50 of torque shaft 48 distal to the injection port 49. A helical coil screw 53 is secured to the distal end 54 of the torque shaft 48 and rotation of the torque knob 52 on the proximal end 50 of the torque shaft 48 rotates the screw 53 on the distal end 54 of torque shaft 48 to facilitate deployment of a partitioning device 10. A inflatable balloon 55 is sealingly secured to the distal end of the inner shaft 43 and has an interior 56 in fluid communication with the first lumen 44. Inflation fluid may be delivered to the interior 56 through port 44a in the portion of the inner shaft 43 extending through the balloon 55. Inflation of the balloon 55 by inflation fluid through port 46 facilitates securing the partitioning component 10.

To deliver the partitioning component 10, it is secured to the distal end of the delivery catheter 32 by means of the helical coil screw 53. The partitioning component 10 is collapsed to a first, delivery configuration which has small enough transverse dimensions to be slidably advanced through the inner lumen 33 of the guide catheter 31. Preferably, the guide catheter 31 has been previously percutaneously introduced and advanced through the patient's vasculature, such as the femoral artery, in a conventional manner to the desired heart chamber. The delivery catheter 32 with the partitioning component 10 attached is advanced through the inner lumen 33 of the guide catheter 31 until the partitioning component 10 is ready for deployment from the distal end of the guide catheter 31 into the patient's heart chamber 58 to be partitioned.

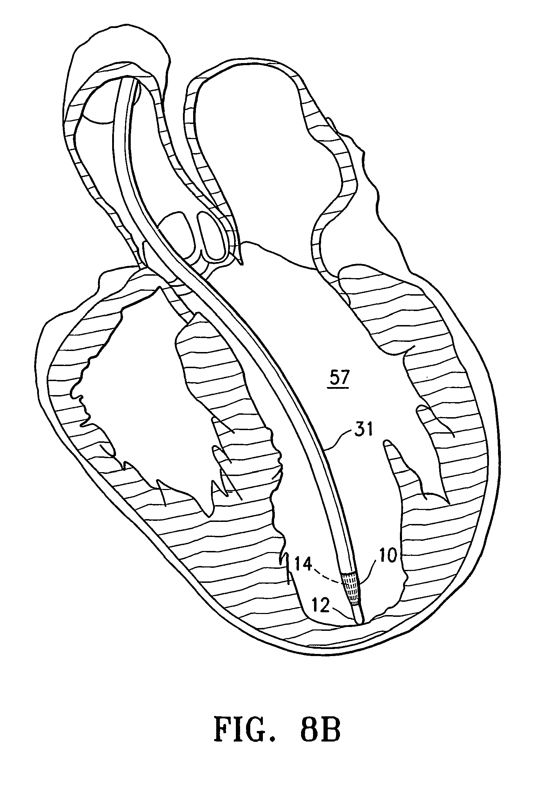

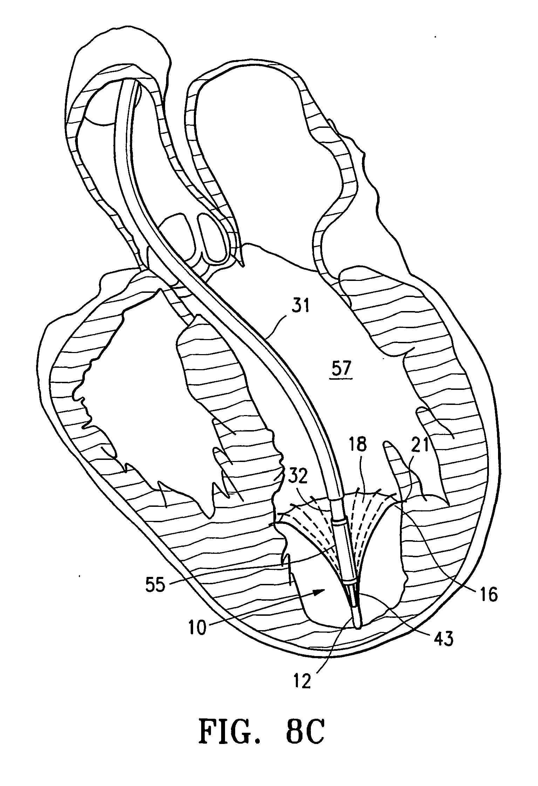

The partitioning component 10 mounted on the screw 53 is urged partially out of the inner lumen 33 of the guide catheter 31 until the hub 12 engages the heart wall as shown in Figure 8B with the free proximal ends 16 of the ribs 14 in a contracted configuration within the guide catheter. The guiding catheter 31 is withdrawn while the delivery catheter 32 is held in place until the proximal ends 16 of the ribs 14 exit the distal end 35 of the guiding catheter. The free proximal ends 16 of ribs 14 expand outwardly to press the sharp proximal tips 21 of the ribs 14 against and preferably into the tissue lining the heart chamber. This is shown in Figure 8C.

With the partitioning component deployed within the heart chamber and preferably partially secured therein, inflation fluid is introduced through the inflation port 46 into first lumen 44 of inner shaft 43 of the delivery catheter 32 where it is directed through port 44a into the balloon interior 56 to inflate the balloon. The inflated balloon presses against the pressure receiving surface 18 of the partitioning component 10 to ensure that the sharp proximal tips 21 are pressed well into the tissue lining the heart chamber.

With the partitioning device 10 properly positioned within the heart chamber, the knob 52 on the torque shaft 48 is rotated counter-clockwise to disengage the helical coil screw 53 of the delivery catheter 32 from the hub 12. The counter-clockwise rotation of the torque shaft 48 rotates the helical coil screw 53 which rides on the connector bar 20 secured within the hub 12. Once the helical coil screw 53 disengages the connector bar 20, the delivery system 30, including the guide catheter 31 and the delivery catheter 32, may then be removed from the patient.

The proximal end of the guide catheter 31 is provided with an flush port 36 to inject therapeutic or diagnostic fluids through the inner lumen 33. Similarly, the proximal end of the delivery catheter 32 is provided with a flush port 47 in communication with inner lumen 41 for essentially the same purpose. An inflation port 46 is provided on the proximal portion of the delivery catheter for delivery of inflation fluid through the first inner lumen 44 to the interior 56 of the balloon 55. An injection port 49 is provided on the proximal end of the torque shaft 48 in fluid communication with the inner lumen 51 of the torque shaft for delivery of a variety of fluids.

The partitioning component 10 partitions the patient's heart chamber 57 into a main productive or operational portion 58 and a secondary, essentially non-productive portion 59. The operational portion 58 is much smaller than the original ventricular chamber 57 and provides for an improved ejection fraction. The partitioning increases the ejection fraction and provides an improvement in blood flow. Over time, the non-productive portion 59 fills first with thrombus and subsequently with cellular growth. Bio-resorbable fillers such as polylactic acid, polyglycolic acid, polycaprolactone and copolymers and blends may be employed to initially fill the non-productive portion 59. Fillers may be suitably supplied in a suitable solvent such as DMSO. Other materials which accelerate tissue growth or thrombus may be deployed in the non-productive portion 59.

Figures 9 and 10 illustrate the reinforcing frame 13 in an unstressed configuration and includes the ribs 14 and the hub 12. The ribs 14 have a length L of about 1 to about 8 cm, preferably, about 1.5 to about 4 cm for most left ventricle deployments. The proximal ends 16 have a flared construction. To assist in properly locating the device during advancement and placement thereof into a patient's heart chamber, parts, e.g. the distal extremity, of one or more of the ribs and/or the hub may be provided with markers at desirable locations that provide enhanced visualization by eye, by ultrasound, by X-ray, or other imaging or visualization means. Radiopaque markers may be made with, for example, stainless steel, platinum, gold, iridium, tantalum, tungsten, silver, rhodium, nickel, bismuth, other radiopaque metals, alloys and oxides of these metals.

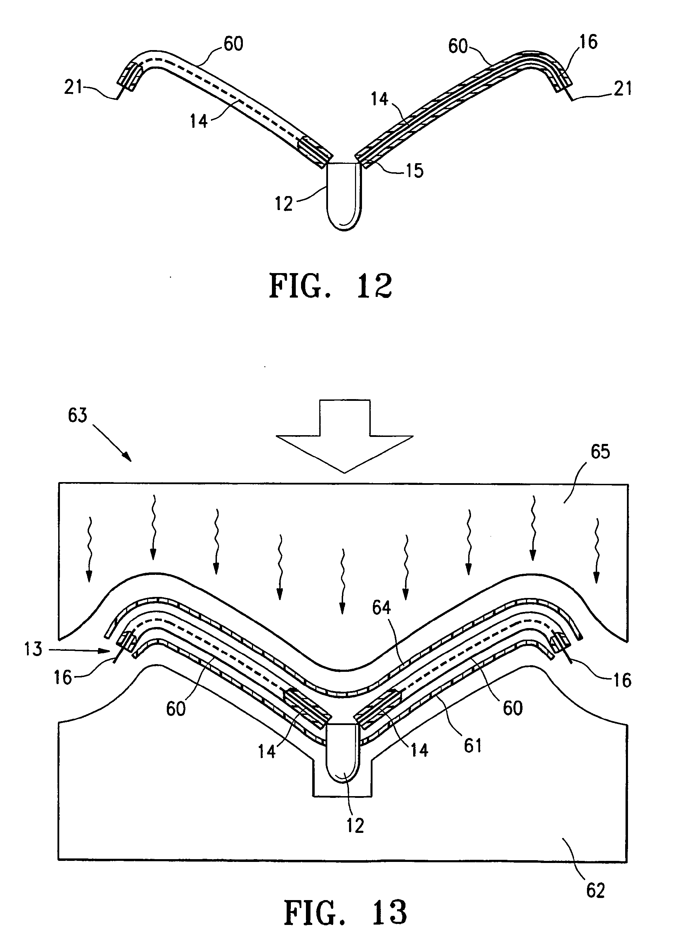

The partitioning device 10 is conveniently formed by placing a thermoplastic tube 60, e.g. polyethylene, over the ribs 14 of the frame 13 as shown in Figure 11 until the proximal ends 16 of the ribs 14 extend out the ends of the thermoplastic tubes as shown in Figure 12. A first expanded PTFE sheet 61 of appropriate size is placed in the female platen 62 of the press 63. The frame 13 with tubes 60 slidably disposed over the ribs 14 is placed in platen 62 on top of the ePTFE sheet 61. The center portion of the sheet 61 may be provided with an opening through which the hub 12 extends. A second ePTFE sheet 64 is placed on top of the ribs 14 of frame 13 as shown in Figure 13. The male platen 65 is heated, preferably to about 500°F, so that the thermoplastic tubes 60 disposed over the ribs 14 fuse into the porous matrix of the ePTFE sheets 61 and 64. The fused thermoplastic material solidifies and secures the sheets 61 and 64 to the ribs 14 and prevents their delamination during use.

While porous ePTFE material is preferred, the membrane 11 may be formed of suitable biocompatitble polymeric material which include Nylon, PET (polyethylene terephthalate) and polyesters such as Hytrel. The membrane 11 is preferably foraminous in nature to facilitate tissue ingrowth after deployment within the patient's heart. The delivery catheter 32 and the guiding catheter 31 may be formed of suitable high strength polymeric material such as PEEK (polyetheretherketone), polycarbonate, PET, Nylon, and the like. Braided composite shafts may also be employed.

To the extent not otherwise described herein, the various components of the partitioning device and delivery system may be formed of conventional materials and in a conventional manner as will be appreciated by those skilled in the art.

While particular forms of the invention have been illustrated and described herein, it will be apparent that various modifications and improvements can be made to the invention. Moreover, individual features of embodiments of the invention may be shown in some drawings and not in others, but those skilled in the art will recognize that individual features of one embodiment of the invention can be combined with any or all the features of another embodiment. Accordingly, it is not intended that the invention be limited to the specific embodiments illustrated. It is intended that this invention to be defined by the scope of the appended claims as broadly as the prior art will permit.

## Claims

1. A device for treating a patient's heart by partitioning a ventricle of the patient's heart into a primary productive portion and a secondary non-productive portion, comprising:
a. a partitioning component (10) which has an expandable frame (13) formed of a plurality of ribs (14) having distal ends (15) secured to a central hub (12) and free outwardly curved proximal ends (16) and which has a proximal, pressure receiving face (18) formed at least in part of a membrane (11) secured to the expandable frame ribs, and forming a recess in an expanded, deployed configuration defining in part the primary productive portion of the patient's heart chamber to be partitioned; and
b. a plurality of tissue penetrating securing elements (21) disposed at free ends of the ribs configured to penetrate tissue lining the heart chamber to secure the periphery of the partitioning device to the heart chamber; and
wherein the hub has a non-traumatic distal tip to engage the region of the patient's ventricular wall.

2. The device of claim 1 wherein the partitioning component (10) has a contracted configuration for delivery to patient's heart chamber to be partitioned.

3. The device of claim 1 wherein the membrane (11) is foraminous.

4. The device of claim 1 wherein the membrane (11) is formed at least in part of a polymeric fabric.

5. The device of claim 1 wherein the distal ends (15) of the ribs (14) are configured to facilitate abduction of the free proximal ends (16) of the ribs away from a centerline axis to facilitate expansion of the partitioning component.

6. The device of claim 1 wherein the free proximal ends (16) of the ribs (14) are outwardly curved.

7. The device of claim 6 wherein the free proximal ends (16) of the ribs (14) have tips which penetrate the tissue lining the heart chamber at an angle of not more than 45° away from a line perpendicular to the center line axis of the partitioning device.

8. The device of claim 1 wherein the pressure receiving face (18) has radial dimensions from a center line axis of about 5 to about 80 mm.

9. The device of claim 1 wherein the pressure receiving face (18) has radial dimensions from a center line axis of about 5 to about 80 mm.

10. The device of claim 1 wherein the frame (13) has about 3 to about 30 ribs.

11. The device of claim 1 wherein the frame (13) has about 6 to about 16 ribs.

12. The device of claim 1 wherein the expandable frame (13) is self expanding.

13. The device of claim 1 wherein the frame (13) is formed of superelastic NiTi alloy which is in an austenite phase when unstressed.

14. The device of claim 1 wherein the frame (13) is in a stress maintained martensite phase when delivered through the patient's vasculature to the patient's heart chamber.

15. The device of claim 1 wherein the membrane (11) is formed at least in part of expanded fluoropolymer.

16. The device of claim 15 wherein the expanded fluoropolymer is polytetrafluoroethylene.

17. The partitioning apparatus of claim 1 wherein the non-traumatic distal tip has a bullet shape.

18. The partitioning apparatus of claim 1 wherein the membrane (11) is secured to the proximal side of the ribs.

19. The partitioning apparatus of claim 1 wherein the membrane (11) is secured to the distal side of the ribs.

## Patentansprüche

1. Vorrichtung zur Behandlung des Herzes eines Patienten durch Partitionierung eines Ventrikels des Herzes des Patienten in einen ersten produktiven Teil und einen zweiten nicht-produktiven Teil, umfassend:
a. eine Partitionierungskomponente (10), die einen expandierbaren Rahmen (13) hat, der aus einer Vielzahl von Rippen (14) gebildet wird, die distale Enden (15), die an einer zentralen Nabe (12) befestigt sind, und freie nach außen gebogene proximale Enden (16) haben, und die eine proximale Druck-aufnehmende Seite (18) hat, die wenigstens teilweise aus einer Membran (11) gebildet wird, die an den expandierbaren Rahmenrippen befestigt ist, und die eine Ausnehmung in einer expandierten, entfalteten Konfiguration bildet, die zum Teil den ersten produktiven Teil der Herzkammer des Patienten, die partitioniert werden soll, bildet, und
b) eine Vielzahl von Gewebe-penetrierenden Befestigungselementen (21), die an freien Enden der Rippen angeordnet sind, die konfiguriert sind, um Gewebe, das die Herzkammer auskleidet, zu penetrieren, um den Außenrand der Partitionierungsvorrichtung an der Herzkammer zu befestigen, und
wobei die Nabe eine nicht-traumatische distale Spitze hat, um in die Region der ventrikulären Wand des Patienten einzutreten.

2. Vorrichtung gemäß Anspruch 1, wobei die Partitionierungskomponente (10) zur Abgabe in die Herzkammer des Patienten, die partitioniert werden soll, eine zusammengezogene Konfiguration hat.

3. Vorrichtung gemäß Anspruch 1, wobei die Membran (11) perforiert ist.

4. Vorrichtung gemäß Anspruch 1, wobei die Membran (11) wenigstens zum Teil aus einem Polymergewebe gebildet ist.

5. Vorrichtung gemäß Anspruch 1, wobei die distalen Enden (15) der Rippen (14) so konfiguriert sind, dass sie eine Führung der freien proximalen Enden (16) der Rippen weg von der Mittellinienachse erleichtern, um eine Expansion der Partitionierungskomponente zu erleichtern.

6. Vorrichtung gemäß Anspruch 1, wobei die freien proximalen Enden (16) der Rippen (14) nach außen gebogen sind.

7. Vorrichtung gemäß Anspruch 6, wobei die freien proximalen Enden (16) der Rippen (14) Spitzen haben, welche das Gewebe, das die Herzkammer auskleidet, in einem Winkel von nicht mehr als 45° von einer Linie aus, senkrecht zu der Mittellinienachse der Partitionierungsvorrichtung, penetrieren.

8. Vorrichtung gemäß Anspruch 1, wobei die Druck-aufnehmende Seite (18) radiale Abmessung ab einer Mittellinienachse von etwa 5 bis etwa 80mm hat.

9. Vorrichtung gemäß Anspruch 1, wobei die Druck-aufnehmende Seite (18) radiale Abmessungenen ab einer Mittellinienachse von etwa 5 bis etwas 80mm hat.

10. Vorrichtung gemäß Anspruch 1, wobei der Rahmen (13) etwa 3 bis etwa 30 Rippen hat.

11. Vorrichtung gemäß Anspruch 1, wobei der Rahmen (13) etwa 6 bis etwa 16 Rippen hat.

12. Vorrichtung gemäß Anspruch 1, wobei der expandierbare Rahmen (13) selbst-expandierend ist.

13. Vorrichtung gemäß Anspruch 1, wobei der Rahmen (13) aus superelastischer NiTi-Legierung geformt ist, welche in einer Austenit-Phase ist, wenn sie ungespannt ist.

14. Vorrichtung gemäß Anspruch 1, wobei der Rahmen (13) in einer durch Spannung aufrecht erhaltenen Martensit-Phase ist, wenn er durch das Gefäßsystem des Patienten an die Herzkammer des Patienten abgegeben ist.

15. Vorrichtung gemäß Anspruch 1, wobei die Membran (11) wenigstens teilweise aus expandiertem Fluorpolymer gebildet ist.

16. Vorrichtung gemäß Anspruch 15, wobei das expandierte Fluorpolymer, Polytetrafluorethylen ist.

17. Partitionierungsapparatur gemäß Anspruch 1, wobei die nicht-traumatische distale Spitze eine Kugelform hat.

18. Partitionierungsapparatur gemäß Anspruch 1, wobei die Membran (11) an der proximalen Seite der Rippen befestigt ist.

19. Partitionierungsappartur gemäß Anspruch 1, wobei die Membran (11) an der distalen Seite der Rippen befestigt ist.

## Revendications

1. Dispositif pour le traitement du coeur d'un patient en divisant un ventricule du coeur du patient en une partie productive principale et une partie secondaire, non productive, comprenant :
a. un composant de séparation (10) qui a une carcasse extensible (13) constituée d'une pluralité de nervures (14) ayant des extrémités distales (15) fixées à un moyeu central (12) et des extrémités proximales libres, courbées vers l'extérieur, (16) et qui a une face proximale recevant une pression (18) formée au moins en partie d'une membrane (11) fixée aux nervures de la carcasse extensible, et formant un évidement dans une configuration déployée et étendue définissant en partie la partie productive principale de la chambre du coeur du patient à diviser ; et
b. une pluralité d'éléments de fixation pénétrant dans un tissu (21) placés aux extrémités libres des nervures, configurés pour pénétrer dans le tissu qui double la chambre du coeur afin de fixer la périphérie du dispositif de séparation à la chambre du coeur ; et
dans lequel le moyeu a un bout distal non traumatique pour le contact avec la région de la paroi ventriculaire du patient.

2. Dispositif selon la revendication 1, dans lequel le composant de séparation (10) a une configuration contractée pour être amené dans la chambre du coeur du patient à diviser.

3. Dispositif selon la revendication 1, dans lequel la membrane (11) est perforée.

4. Dispositif selon la revendication 1, dans lequel la membrane (11) est formée au moins en partie d'un tissu en polymère.

5. Dispositif selon la revendication 1, dans lequel les extrémités distales (15) des nervures (14) sont configurées pour faciliter l'abduction des extrémités proximales libres (16) des nervures à l'écart d'un axe central pour faciliter l'extension du composant de séparation.

6. Dispositif selon la revendication 1, dans lequel les extrémités proximales libres (16) des nervures (14) sont courbées vers l'extérieur.

7. Dispositif selon la revendication 6, dans lequel les extrémités proximales libres (16) des nervures (14) ont des bouts qui pénètrent dans le tissu qui double la chambre du coeur en formant un angle qui ne dépasse pas 45° par rapport à une droite perpendiculaire à l'axe central du dispositif de séparation.

8. Dispositif selon la revendication 1, dans lequel la face recevant une pression (18) a des dimensions radiales à partir d'un axe central qui valent d'environ 5 à environ 80 mm.

9. Dispositif selon la revendication 1, dans lequel la face recevant une pression (18) a des dimensions radiales à partir d'un axe central qui valent d'environ 5 à environ 80 mm.

10. Dispositif selon la revendication 1, dans lequel la carcasse (13) comporte d'environ 3 à environ 30 nervures.

11. Dispositif selon la revendication 1, dans lequel la carcasse (13) comporte d'environ 6 à environ 16 nervures.

12. Dispositif selon la revendication 1, dans lequel la carcasse extensible (13) est auto extensible.

13. Dispositif selon la revendication 1, dans lequel la carcasse (13) est faite d'un alliage NiTi superélastique qui est dans une phase austénitique quand il n'est pas sous contrainte.

14. Dispositif selon la revendication 1, dans lequel la carcasse (13) est dans une phase martensitique maintenue sous contrainte quand elle est administrée dans la chambre du coeur du patient via le système vasculaire du patient.

15. Dispositif selon la revendication 1, dans lequel la membrane (11) est faite au moins en partie de fluoropolymère expansé.

16. Dispositif selon la revendication 15, dans lequel le fluoropolymère expansé est du polytétrafluoroéthylène.

17. Dispositif de séparation selon la revendication 1, dans lequel le bout distal non traumatique a une forme de balle (projectile).

18. Dispositif selon la revendication 1, dans lequel la membrane (11) est fixée sur le côté proximal des nervures.

19. Dispositif selon la revendication 1, dans lequel la membrane (11) est fixée sur le côté distal des nervures.
